Europäisches Patentamt

(19) European Patent Office

Office européen des brevets

(11) Publication number : **0 453 891 B1**

# EUROPEAN PATENT SPECIFICATION

(45) Date of publication of patent specification : **01.06.94 Bulletin 94/22**

(51) Int. Cl.[5] : **C07C 21/18, C07C 17/26**

(21) Application number : **91105773.5**

(22) Date of filing : **11.04.91**

(54) **Preparation of 3,3,3-trifluoropropene-1.**

(30) Priority : **12.04.90 US 508680**

(43) Date of publication of application : **30.10.91 Bulletin 91/44**

(45) Publication of the grant of the patent : **01.06.94 Bulletin 94/22**

(84) Designated Contracting States : **DE FR GB**

(56) References cited :
DE-A- 3 823 370
GB-A- 772 484
GB-A- 979 618
US-A- 4 220 608

(56) References cited :
CHEMICAL ABSTRACTS, vol. 90, no. 11, 12 March 1979, Columbus, Ohio, US; A. E. FEIRING, "Synthesis of 3,3,3-trifluoropropene from vinylidene fluoride", page 552, abstract no. 86 653h
CHEMICAL ABSTRACTS, vol. 105, no. 11, 15 September 1986, Columbus, Ohio, US; M. HISAZUMI, "Synthesis of fluororesin monomers. VII. Dilution effects for the formation of 3,3,3-trifluoropropene in the quick copyrolysis of bromotrifluoromethane and ethene", page 581, abstract no. 96 879d

(73) Proprietor : **DOW CORNING CORPORATION**
P.O. Box 1767
**Midland Michigan 48686-0994 (US)**

(72) Inventor : **Bajzer, William Xavier**
**4201 McKeith Road**
**Midland, Michigan (US)**

(74) Representative : **Spott, Gottfried, Dr. et al**
**Spott Weinmiller & Partner**
**Sendlinger-Tor-Platz 11**
**D-80336 München (DE)**

EP 0 453 891 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 453 891 B1

## Description

The instant invention is an isomer-specific process for the preparation of 3,3,3-trifluoropropene-1. The process comprises co-feeding a difluorocarbene precursor and a vinyl fluoride monomer to a reactor at a temperature above the degradation temperature of the difluorocarbene precursor, but lower than 800°C. When the residence time of the feed materials and resultant products within the reactor is within the range of one to 10 seconds, the process is isomer specific for 3,3,3-trifluoropropene-1.

3,3,3-Trifluoropropene-1 (TFP) is a chemical intermediate that has significant value as a commercial raw material. A primary example of the commercial value of TFP is its use in the preparation of fluorosilicone intermediates and such final products as fluorosilicone fluids and fluorosilicone rubber.

Industrially, TFP is typically produced by reacting carbon tetrachloride with ethylene to create the intermediate compound 1,1,1,3-tetrachloropropane. This intermediate compound is then reacted with excess hydrogen fluoride at elevated temperature, in the presence of a catalyst, to produce TFP. The recovery and recycling of the excess hydrogen fluoride, due to its corrosive nature, is expensive and cumbersome. Therefore, an alternative to this process is desirable.

The inventors have discovered that vinyl fluoride can effectively compete with the reaction of difluorocarbene to form TFE, to form the product 3,3,3-trifluoropropene-1. This reaction is isomer-specific and can be ran as a one-step process without the need for hydrogen fluoride.

The instant invention is a isomer-specific process for the preparation of 3,3,3-trifluoropropene-1. The process comprises co-feeding a difluorocarbene precursor and vinyl fluoride monomer to a reactor. The reactor is at a temperature sufficient to decompose the difluorocarbene precursor to difluorocarbene and less than 800°C. The residence time of the feed materials and products thereof within the reactor is 1.0 to 10 seconds.

Difluorocarbene is an extremely reactive material that preferentially reacts with itself to form oligmers of the structure $-(CF_2)_x-$, where x is two or more. To minimize this reaction, a preferred embodiment of the instant invention is a one-step process where a precursor compound, which is thermally degradable into a difluorocarbene component, is a co-feed with vinyl fluoride monomer to a heated reactor. As the co-feed gases pass through the elevated temperature of the reactor, the precursor compound degrades to a difluorocarbene free radical component which can react with the vinyl fluoride to form the 3,3,3-trifluoropropene-1 isomer (TFP). The process is isomer specific. By isomer specific is meant, that greater than 99 percent of the product propene is 3,3,3-trifluoropropene-1.

As an alternative to the preferred approach, the difluorocarbene can be generated in a separate process and then co-fed with the vinyl fluoride monomer.

By "co-feed" is meant, the feed materials are either mixed prior to entry into the reactor or are both fed simultaneously into the same end of the reactor.

The precursor difluorocarbene compound can be diluted with an inert gas, for example, helium, nitrogen or argon or with steam. Dilution of the precursor compound can suppress by-product oligomer formation.

The reactor can be any standard apparatus for contacting gases at elevated temperatures. The reactor could be, for example, a heated quartz glass or stainless steel tube with inlet and exit ports. In addition, the reactor could contain an inert particulate fluid or packed bed. By inert, is meant any particulate material that does not effect the product distribution. Since hydrogen chloride is typically a byproduct of the described process it is preferred the reactor be constructed of corrosion resistant materials such as stainless steel or quartz glass.

The reactor temperature can be any temperature above the temperature at which the difluorocarbene precursor decomposes to form a difluorocarbene free radical. However, when the temperature of the reactor is above about 800°C., there is a tendency for feed materials to carbonize on the reactor. Although this problem could possibly be reduced by shorter residence times within the reactor, no advantage is seen in attempting to run the reactor at temperatures above 800°C. If the difluorocarbene is generated external to the reactor and then co-fed with the vinyl fluoride, a preferred temperature of the reactor is 100°C. to 800°C.

The difluorocarbene precursor can be, for example, trifluoromethane or a halodifluoromethane, such as chlorodifluoromethane, bromodifluoromethane or iododifluoromethane. The difluorocarbene precursor can be, for example, tetrafluoroethylene monomer or poly(tetrafluoroethylene). A preferred difluorocarbene precursor is chlorodifluoromethane.

When the difluorocarbene precursor is a co-feed with the vinyl fluoride monomer, the reactor temperature must be at a temperature above the temperature at which the precursor compound degrades to form difluorocarbene. When the precursor compound is chlorodifluoromethane, tetrafluoroethylene or poly(tetrafluoroethylene) a useful temperature range is 550°C. to 800°C. A preferred temperature range, for the reactor, with these three precursor compounds is 625°C. to 730°C. When the precursor compound is bromodifluoromethane, a useful temperature range is 200°C. to 800°C. When the precursor compound is iodo-

2

difluoromethane, a useful temperature range is 100°C. to 800°C. The preferred temperature range for bromodifluoromethane and iododifluoromethane is 200°C. to 400°C.

The inventors believe the difluorocarbene reacts with the vinyl fluoride monomer according to the following equation:

$$CF_2: + CH_2=CHF \rightarrow CF_3CH=CH2$$

Therefore, theoretically, stoichiometric equivalents of the difluorocarbene and vinyl fluoride monomer should be desirable. In practice, a stiochiometric ratio of vinyl fluoride to difluorocarbene precursor in the range of 0.2 to 5.0 has been found useful. For the purpose of the instant invention, a ratio of vinyl fluoride to difluorocarbene in the range of 1.0 to 1.5 is preferred.

The highly reactive difluorocarbene reacts with the vinyl fluoride monomer quickly. However, the inventors theorize, that the quantative transfer of the fluoride atom from the carbon on the vinyl fluoride monomer to the three position of the 3,3,3-trifluoropropene-1 molecule may require additional time at reactor temperatures. In practice, it has been found that the total reactor residence time for the vinyl fluoride and difluorocarbene precursor to be converted into 3,3,3-tifluoropropene-1 is 1.0 to 10 seconds. When the thermally degradable precursor is chlorodifluoromethane, a residence time of 1.4 to 3.6 seconds is preferred.

So that those skilled in the art can better appreciate and understand the present invention, the following examples are given to be illustrative and are not to be construed as limiting the instant invention as described herein.

Example 1

Vinyl fluoride (VFM), $CH_2=CHF$, was purchased from PCR Research Chemicals, Inc., Gainesville, Florida, and supplied in cylinders with added d-limonene as inhibitor. R-22 refrigerant gas, chlorodifluoromethane, was obtained in a cylinder from Matheson Gas Products, Secaucus, NJ. All gases were metered through individual rotameters and then mixed in a mixing header. The combined streams were directed into a quartz tube 12 mm o.d. x 10 mm i.d., inserted in a tube furnace. A calculated heated reactor volume of 15.96 cc was based on the 20,3 cm (eight inch) long heated zone of the quartz reactor within the 30,5 cm (12 inch) long tube furnace. Gas flow rates were divided into the 15.96 cc volume to calculate the contact time in the furnace. The temperature was measured externally to the reactor tube in the heated zone. R-22 was pyrolyzed for about two hours in the reactor tube as a preconditioning step prior to experimental work.

The vent gases were directed through teflon tubing to a dry ice trap. A small glass tee near the dry ice trap was fitted with a septum so gas samples could be acquired for gas chromotography analysis (GC). The conversion of feed and the yield of product were calculated from the GC area analyses.

A number of runs were conducted where temperature, mole ratio of reactants and contact time was varied. The results are shown in Table 1. The heading "Ratio VFM/R22" refers to the molar feed ratio of vinyl fluoride monomer (VFM) to chlorodifluoromethane (R22) for the temperature and contact time condition indicated. In each run, a variable amount of unreacted VFM and R22 are detected in the reactor effluent stream. The heading "% CONV R22" refers to the percent of R22 consumed in the reaction zone to form tetrafluoroethylene monomer (TFEM), TFP and by-products of the thermal degradation of R22. The headings "% TFEM" and "%TFP" refer to the percent yields of TFEM and TFP, respectively, based on converted R22. The remainder between the sum of these two values and 100 percent is attributable to by-product formation. The by-products are known in the literature to be oligomeric compounds of the structure $H(CF_2)Cl$ and TFEM dimer ($C_4F_8$), amoung others.

## Table 1

### Conversion of Chlorodifluoromethane to 3,3,3-Trifluoropropene

| Run | Temp. (°C.) | Ratio VFM/R22 | Contact time(Sec.) | %Conv. R22 | % TFEM | % TFP |
|-----|-------------|---------------|---------------------|------------|--------|-------|
| D | 620 | 1.3 | 3.6 | 64 | 90 | 2.3 |
| E | 620 | 1.3 | 3.6 | 62 | 89 | 2.2 |
| J | 670 | 1.3 | 3.6 | 85 | 77 | 4.1 |
| K | 695 | 1.3 | 3.6 | 90 | 56 | 5.6 |
| L | 720 | 1.3 | 3.6 | 96 | 24 | 6.8 |
| M | 720 | 0.6 | 2.3 | 90 | 55 | 2.9 |
| N | 710 | 0.3 | 1.4 | 75 | 83 | 0.8 |
| O | 625 | 1.3 | 3.6 | 53 | 96 | 2.1 |
| P | 725 | 1.3 | 3.6 | 91 | 41 | 6.7 |
| R | 720 | 1.3 | 3.6 | 92 | 40 | 6.8 |

Analytical characterization using infrared spectroscopy (IR), nuclear magnetic resonance spectrometry (NMR) and gas chromatography with mass spectrometry (GC-MS) all confirm the sole $C_3H_3F_3$ isomer as exclusively the 3,3,3-trifluoropropene structure. The data in Table 1 indicate that conversion of chlorodifluoromethane to TFP increases with increase in temperature. However, it was observed that at temperatures above about 800°C. significant carbon buildup occurred on the walls of the reactor. Therefore, 800°C. appears to be the practical upper limit for temperature, under flow conditions as described. Conversion of chlorodifluoromethane to TFP also increased with increases in contact time.

## Claims

1.  An isomer specific process for preparation of 3,3,3-trifluoropropene-1, the process comprising co-feeding a difluorocarbene precursor and vinyl fluoride monomer to a reactor; for a residence time of one to 10 seconds; at a reactor temperature sufficient to decompose the difluorocarbene precursor to difluorocarbene and less than 800°C.

2.  A process according to claim 1 were the vinyl fluoride monomer is co-fed at a stoichiometric ratio of 0.2 to 5.0 in relation to the difluorocarbene precursor.

3.  An isomer specific process comprising preparation of 3,3,3-trifluoropropene-1 by co-feeding difluorocarbene and vinyl fluoride monomer to a reactor; for a residence time of one to 10 seconds; at a reactor temperature of 100°C. to 800°C.

## Patentansprüche

1.  Isomerspezifisches Verfahren zur Herstellung von 3,3,3-Trifluorpropen-1, wobei das Verfahren umfaßt, daß man einen Difluorcarbenvorläufer und Vinylfluoridmonomer gleichzeitig in einen Reaktor führt bei einer Verweilzeit von 1 bis 10 Sekunden, bei einer Reaktortemperatur, die ausreicht, um den Difluorcarbenvorläufer zu Difluorcarben zu zersetzen und geringer als 800°C ist.

2.  Verfahren nach Anspruch 1, worin das Vinylfluoridmonomer in einem stöchiometrischen Verhältnis von 0,2 bis 5,0 bezogen auf den Difluorcarbenvorläufer zugeführt wird.

3.  Isomerspezifisches Verfahren, umfassend die Herstellung von 3,3,3-Trifluorpropen-1 durch gleichzeitige Zuführung von Difluorcarben und Vinylfluoridmonomer in einen Reaktor mit einer Verweilzeit von 1 bis 10 Sekunden und bei einer Reaktortemperatur von 100 bis 800°C.

**Revendications**

1. Procédé spécifique à un isomère pour la préparation de 3,3,3-trifluoropropène-1, ce procédé comprenant la co-introduction d'un précurseur du difluorocarbène et de monomère fluorure de vinyle dans un réacteur ; pendant un temps de séjour d'une à 10 seconde(s) ; à une température du réacteur suffisante pour décomposer le précurseur du difluorocarbène en difluorocarbène, et inférieure à 800 °C.

2. Procédé selon la revendication 1, dans lequel le monomère fluorure de vinyle est co-introduit à un rapport stoechiométrique de 0,2 à 0,5 par rapport au précurseur du difluorocarbène.

3. Procédé spécifique à un isomère, comprenant la préparation de 3,3,3-trifluoropropène-1 en co-introduisant du difluorocarbène et du monomère fluorure de vinyle dans un réacteur ; pendant un temps de séjour d'une à 10 seconde(s) ; à une température du réacteur de 100 °C à 800 °C.